# EUROPÄISCHE PATENTSCHRIFT

(11) **EP 0 714 872 B1**
(45) Veröffentlichungstag und Bekanntmachung des Hinweises auf die Patenterteilung: **16.08.2000**
(21) Anmeldenummer: 95117393.9
(22) Anmeldetag: 04.11.1995
(51) Int. Cl.: C07C 5/333, C07C 11/02, B01J 23/62

(54) **Katalysator für die Dehydrierung von C6-bis C15-Paraffinen**
Catalyst for the dehydrogenation of C6-C15 paraffins
Catalyseur pour la déshydrogénation de paraffines C6-C15

(30) Priorität: 29.11.1994 DE 4442327
(43) Veröffentlichungstag der Anmeldung: 05.06.1996
(73) Patentinhaber: Degussa-Hüls Aktiengesellschaft, 60287 Frankfurt am Main (DE)
(72) Erfinder: Lansink-Rotgerink, Hans, Dr., D-63864 Glattbach (DE); Tacke, Thomas, Dr., D-61381 Friedrichsdorf (DE); Brand, Reinhold, Dr., D-63571 Gelnhausen (DE); Panster, Peter, Dr., D-63517 Rodenbach (DE)

(56) Entgegenhaltungen:
- BE-A- 753 072
- US-A- 3 998 900
- US-A- 4 886 928
- US-A- 4 964 975

## Beschreibung

Die Erfindung betrifft einen Katalysator für die Dehydrierung von C₆- bis C₁₅-Paraffinen.

Die Dehydrierung von Kohlenwasserstoffen und insbesondere von Paraffinen wird im großtechnischen Maßstab durchgeführt. Die dehydrierten Produkte werden für die Herstellung verschiedener chemischer Verbindungen benötigt, wie zum Beispiel Detergentien, Benzinkomponenten und pharmazeutische Produkte. Von besonderer Bedeutung für die Herstellung von Detergentien ist die Dehydrierung von normalen Paraffinen mit 6 - 15 Kohlenstoffatomen zu den entsprechenden Monoolefinen. Die Monoolefine werden für die Alkylierung von aromatischen Verbindungen wie zum Beispiel Benzol, zu den entsprechenden linearen Alkylbenzolen benötigt.

Die Dehydrierung der Paraffine wird an einem geeigneten Katalysator unter Dehydrierbedingungen vorgenommen. Die Kontaktierung der Paraffine mit dem Katalysator kann in einem Festbett, in einem Fließbett oder in einem Wirbelbett durchgeführt werden. Bevorzugt wird ein Festbettkatalysatorsystem eingesetzt. Hierzu wird das zu dehydrierende Edukt zunächst auf die gewünschte Reaktionstemperatur vorgeheizt und dann über den Festbettkatalysator geführt. Bevorzugt werden die Kohlenwasserstoffe in der Dampfphase dehydriert. Die hierfür erforderlichen Temperaturen liegen im Bereich zwischen 300 und 900°C.

Die für die Dehydrierung im großtechnischen Maßstab eingesetzten Katalysatoren enthalten Platin auf Aluminiumoxid als Trägermaterial. Im allgemeinen enthalten die Katalysatoren noch mehrere Promotoren, zum Beispiel Zinn und/oder Indium. Bei der Dehydrierung von normalen Paraffinen ist es das Ziel, geradkettige Monoolefine herzustellen. Es sollten daher Neben- oder Folgereaktionen zu Isoolefinen, Isoparaffinen oder alkylierten Aromaten vermieden werden. Diese unerwünschten Reaktionen werden in der Praxis durch Zugabe eines Alkalimetalls unterdrückt, das die katalytische Aktivität in geeigneter Weise modifiziert.

Die US-A 4,964,975 beschreibt ein Verfahren zum Umwandeln von Kohlenwasserstoffen, bei welchem als Katalysator ein anorganischer Träger mit Halogen, ein Platingruppenmetall und Zinn eingesetzt wird. Zusätzlich kann der Träger ein Alkali- oder Erdalkalimetall enthalten.

Die US-A 3,998,900 beschreibt ein Verfahren zur Dehydrierung von Kohlenwasserstoffen, bei welchem ein Katalysator eingesetzt wird, bei dem eine Platin/Zinnlösung auf einen porösen Träger aufgetragen und nach dem Trocknen eine Alkali- oder Erdalkalilösung aufgetragen und anschließend in einer sauerstoffhaltigen Atmosphäre kalziniert wird.

Die US-A 4,886,928 beschreibt ein Verfahren zur Dehydrierung von Kohlenwasserstoffen, bei welchem ein Katalysator eingesetzt wird, der eine Komponente aus der Gruppe der seltenen Erden, eine Komponente aus der Gruppe Zinn, Blei und Germanium sowie gegebenenfalls ein Alkali- oder Erdalkalimetall enthält.

Die US-Patentschrift 4,486,547 beansprucht zum Beispiel eine Katalysatorzusammensetzung, die eine Platingruppenkomponente, eine Zinnkomponente, eine Indiumkomponente, eine Alkali- oder Erdalkalikomponente auf einem porösen Trägermaterial enthält. In den Beispielen werden nur Katalysatoren mit dem Alkalimetall Lithium beschrieben.

Durch die zusätzliche Indiumkomponente zeigt das Katalysatorsystem von US-PS 4,486,547 eine höhere Stabilität der Katalysatoraktivität als vergleichbare Katalysatoren ohne Indium. Für den großtechnischen Einsatz solcher Katalysatorsysteme sind jedoch weitere Verbesserungen der Katalysatoraktivität und insbesondere der Langzeitstabilität des Katalysatorsystems wünschenswert.

Es ist daher Aufgabe der vorliegenden Erfindung, ein Katalysatorsystem für die Dehydrierung von normalen Paraffinen zur Verfügung zu stellen, welches sich gegenüber den bisher bekannten Systemen durch eine weiter verbesserte Langzeitstabilität der Katalysatoraktivität auszeichnet.

Diese Aufgabe wird durch einen Katalysator für die Dehydrierung von C₆- bis C₁₅-Paraffinen gelöst, der auf einem anorganischen Trägermaterial 0,01 bis 5 Gew.-% - mindestens eines Platingruppenmetalis, 0,01 bis 5 Gew.-% mindestens eines der Elemente Zinn, Germanium oder Blei als Promotoren sowie Magnesium als zusätzlichen Modifikator mit einem Gewichtsanteil am fertigen Katalysator von 0,01 bis 20 Gew.-% wobei der Träger ein Schüttgewicht von 0,25 bis überhalb von 0,5 g/cm³ aufweist.

Überraschend hat sich gezeigt, daß durch Austausch der im Stand der Technik als Modifikatoren ausschließlich realisierten Alkalimetalle gegen Magnesium die Katalysatorstabilität wesentlich erhöht werden kann.

Das Trägermaterial für die erfindungsgemäßen Katalysatoren sollte ein Porenvolumen zwischen 0,5 und 3 ml/g und eine spezifische Oberfläche, gemessen durch Stickstoffadsorption nach DIN 66 132, von wenigstens 5 m²/g aufweisen. Es muß ausreichend temperaturbeständig gegenüber den bei der Dehydrierung angewendeten Bedingungen sein. Geeignet sind alle Trägermaterialien, die auch schon im Stand der Technik für Dehydrierkatalysatoren eingesetzt werden, wie zum Beispiel Aktivkohle, Kieselsäure, Siliziumcarbid, Tone und verschiedene Silikate. Besonders geeignet sind temperaturbeständige anorganische Oxide wie Aluminiumoxid, Titandioxid, Zirkondioxid, Chromoxid und andere. Bevorzugt wird als Trägermaterial jedoch ein Aluminiumoxid der Übergangsreihe der kristallographischen Phasen, wie zum Beispiel η- oder γ-Aluminiumoxid, eingesetzt.

Das Trägermaterial kann zu Kugeln, Pillen, Extrudaten oder Granalien verformt sein. Auch der Einsatz von pulverförmigem Material ist möglich. Bevorzugt werden jedoch Kugeln aus η- oder γ-Aluminiumoxid mit Durchmessern zwischen 1 und 4 mm eingesetzt.

Das Schüttgewicht dieser Kugeln liegt aufgrund der hohen Porosität des Trägermaterials unterhalb von 0,5 g/cm³, bevorzugt in einem Bereich zwischen 0,25 und 0,4 g/cm³. Unterhalb eines Schüttgewichtes von 0,25 g/cm³ sind die Träger wegen ihrer großen Porosität nicht mehr ausreichend stabil. Oberhalb eines Schüttgewichtes von 0,4 g/cm³ verschlechtert sich für die Reaktanden wegen der geringeren Porosität die Zugänglichkeit der Reaktanden zu den katalytisch aktiven Komponenten.

Als Platingruppenelement für den erfindungsgemäßen Katalysator eignen sich Platin, Palladium, Iridium, Rhodium, Osmium oder Ruthenium oder Mischungen davon. Platin wird jedoch bevorzugt als Platingruppenelement eingesetzt. Die Platingruppenkomponente liegt im gesamten Katalysator feinverteilt vor. Der Anteil der Platingruppenkomponente am Gesamtgewicht des Katalysators sollte zwischen 0,01 bis etwa 5 Gew.-% liegen. Bevorzugt wird ein Gehalt an Platingruppenkomponenten von 0,1 bis 3 Gew.-% verwendet.

Die Platingruppenkomponente kann schon bei der Herstellung des Trägermaterials durch Copräzipitation oder Cogelierung eingebracht werden. Bevorzugt wird die Platingruppenkomponente jedoch durch Imprägnieren in das Trägermaterial eingebracht. Dazu wird eine Lösung oder eine Suspension einer zersetzbaren Vorläuferverbindung des Platingruppenelementes verwendet. Für Platin eignet sich hierfür zum Beispiel eine Lösung von Hexachloroplatinsäure, die durch andere Säuren noch weiter angesäuert sein kann, um eine homogene Durchimprägnierung des gesamten Trägerteilchens zu garantieren. Auch andere Pt-Salze wie zum Beispiel Tetraamminplatin(II)-nitrat, Tetraamminplatin(II)-hydroxid und Tetraamminplatin(II)-chlorid sind als Platinvorstufen geeignet.

Als Promotoren können die Elemente Zinn, Germanium oder Blei einzeln oder in Mischung eingesetzt werden. Bevorzugt wird jedoch Zinn verwendet. Ebenso wie die Platingruppenkomponente ist auch die Zinnkomponente homogen über das Katalysatorpartikel verteilt. Der Gewichtsanteil des Zinns an fertigen Katalysator beträgt 0,01 bis etwa 5 Gew.-%, bevorzugt wird ein Gehalt von 0,1 bis 3,0 Gew.-% angewendet.

Auch die Zinnkomponente kann schon bei der Herstellung des Trägermaterials eingebracht werden. Bevorzugt ist aber auch hier die nachträgliche Imprägnierung mit einer Lösung von Zinnchlorid oder einem anderen Zinnsalz.

Als Modifikatormetall wird erfindungsgemäß Magnesium verwendet. Auch das Erdalkalimetall kann schon während der Herstellung des Trägermaterials eingebracht werden. Bevorzugt ist jedoch die nachträgliche Einbringung durch Imprägnierung mit einer löslichen Erdalkalimetallverbindung. Der Anteil des Erdalkalimetalls am Gesamtgewicht des fertigen Katalysators sollte zwischen 0,01 und 20 Gew.-% liegen. Bevorzugt wird ein Gehalt von 0,1 bis 10 Gew.-% Magnesium eingesetzt.

Ein weiterer Gegenstand der Erfindung ist ein Verfahren zur Herstellung des Katalysators, welches gekennzeichnet ist durch gleichzeitiges Einbringen von löslichen Vorstufen der Platingruppenmetalle, der Promotoren und der Modifikatoren aus einer gemeinsamen Imprägnierlösung in den Träger, Trocknen des Trägers, Calcinieren bei Temperaturen zwischen 300 und 700 °C und abschließendes Reduzieren in einem Wasserstoff enthaltenden Gas bei Temperaturen zwischen 200 und 700 °C, wobei als Modifikator Magnesium mit einem Gewichtsanteil von 0,01 bis 20 Gew.-% am fertigen Katalysator eingesetzt wird.

In einer Ausführungsform kann die gemeinsame Imprägnierlösung noch lösliche Vorstufen von Indium enthalten. Die gemeinsame Imprägnierlösung kann weiterhin noch lösliche Vorstufen von Schwefel enthalten.

Die Platingruppenkomponente, der Promotor sowie auch die Erdalkalikomponente können gleichzeitig oder nacheinander in beliebiger Reihenfolge durch Imprägnieren in das Trägermaterial eingebracht werden. Als besonders vorteilhaft hat es sich erwiesen, alle Komponenten des Katalysators gleichzeitig aus einer gemeinsamen Lösung auf dem Katalysatorträger bzw. Trägermaterial abzuscheiden. Für die Herstellung der gemeinsamen Imprägnierlösung eignet sich als Vorstufe von zum Beispiel Platin ein Tetraamminplatin(II)-salz. Bei Verwendung von Hexachloroplatinsäure in der gemeinsamen Imprägnierlösung kommt es nach kurzer Zeit zu Ausfällungen, die nur durch starkes Ansäuern mit anorganischen Säuren wie Salzsäure oder Salpetersäure verhindert werden können. Wird ein Platintetraammin(II)-salz als Platin-Vorstufe benutzt, so läßt sich die gemeinsame Imprägnierlösung mit geringeren Säurezugaben über lange Zeit stabil halten.

Katalysatoren, die mit Hilfe der einstufigen Imprägnierung hergestellt werden, zeichnen sich durch eine besonders gute Langzeitstabilität ihrer katalytischen Aktivität aus. Darüber hinaus stellt die einstufige Imprägnierung auch eine wesentliche Vereinfachung des Herstellungsverfahrens dar.

Als weiteren Promotor kann der Katalysator auch Indium enthalten. Das Indium hat unter anderem einen positiven Einfluß auf die Langzeitstabilität des Katalysators. Es wird bevorzugt als lösliche Vorstufe zusammen mit den anderen Komponenten des Katalysators durch Imprägnieren in einer Menge von bis zu 5 Gew.-%, bezogen auf das Gesamtgewicht des fertigen Katalysators, eingebracht.

Zusätzlich zu den beschriebenen Komponenten kann der Katalysator noch bis zu 5, bevorzugt bis zu 2 Gew.-% Schwefel enthalten. Der Schwefel kann in jeder geeigneten Form auf den Katalysator aufgebracht werden. Geeignete Schwefelquellen sind zum Beispiel Schwefelwasserstoff sowie Mercaptanverbindungen. Schwefelhaltige Komponenten können auch bereits bei der Trägerherstellung als Sulfate, Sulfite, Sulfide oder als organische Schwefelverbindung eingebracht werden. Bevorzugt ist aber auch im Falle von Schwefel, daß das Trägermaterial mit dem Schwefel zusammen mit den anderen Komponenten des Katalysators aus einer gemeinsamen Imprägnierlösung belegt wird.

Langzeitmessungen mit den erfindungsgemäßen Katalysatoren zeigen, daß sie eine bessere Langzeitstabilität als bekannte Katalysatoren aufweisen, was auf die Verwendung von Magnesium als Modifikator zurückgeführt werden kann.

Nach dem Einbringen der Komponenten in den Träger wird die so erhaltene Katalysatorvorstufe getrocknet und für eine Zeitdauer von 0,5 bis 24 Stunden bei Temperaturen zwischen 300 bis 700°C calciniert. Abschließend wird der calcinierte Katalysator durch eine Gasphasenreduktion mit einem H₂ enthaltendem Gas bei Temperaturen zwischen 200 und 700°C reduziert. Die Reduktionsbedingungen werden so gewählt, daß der überwiegende Anteil der Platingruppenkomponente bis zum Metall reduziert wird. Die Reduktion kann sowohl außerhalb sowie auch innerhalb des Dehydrierreaktors erfolgen.

Der erfindungsgemässe Katalysator kann zur Dehydrierung von C₆ bis C₁₅-Paraffinen eingesetzt werden.

Die Dehydrierung der normalen Paraffine wird bevorzugt in der Gasphase vorgenommen. Dazu werden die Paraffine vor dem Eintritt in die Katalysatorzone auf Temperaturen zwischen 300 und 900°C aufgeheizt und dann bei Drucken zwischen 0,08 und 10 MPa über den Katalysator geführt. Die LHSV (LHSV = liquid hourly space velocity) beträgt dabei bevorzugt zwischen 0,1 und 100 h⁻¹. Der die Dehydrierzone verlassende Produktstrom enthält hauptsächlich unkonvertierte Paraffine, Wasserstoff und Monoolefine. Der Produktstrom wird gewöhnlich gekühlt. Nach Abtrennung einer wasserstoffreichen Gasphase verbleibt eine kohlenwasserstoffreiche flüssige Phase. Das flüssige Produktgemisch kann in eine paraffinreiche und in eine olefinreiche Fraktion aufgeteilt werden. Die paraffinreiche Fraktion wird gewöhnlich zur weiteren Dehydrierung erneut durch den Dehydrierreaktor geleitet, während die olefinreiche Fraktion zur Herstellung von Folge- und Endprodukten genutzt wird. Alternativ kann das gesamte Flüssigprodukt zur Alkylierung von Benzol eingesetzt werden. Hierbei werden die Olefine mit dem Benzol zu linearem Alkylbenzol umgesetzt, welches sich leicht von den unreaktiven Paraffinen trennen läßt. Die Paraffine werden nach der Abtrennung erneut dem Dehydrierreaktor zugeführt. Zwischen dem Dehydrier- und Alkylierungsreaktor können die eventuell im Flüssigprodukt enthaltenen Diolefine selektiv zu Monoolefinen hydriert werden.

Die folgenden Beispiele sollen die Herstellung des erfindungsgemäßen Katalysators sowie seine Verwendung bei der Dehydrierung von linearen Paraffinen weiter erläutern.

### Vergleichsbeispiel 1

Es wurde ein konventioneller Dehydrierkatalysator A mit Lithium als Modifikator hergestellt. Als Trägermaterial wurde ein kugelförmiges Aluminiumoxid mit einem Schüttgewicht von 0,3 g/cm³ und einem Porenvolumen von 1,4 ml/g eingesetzt.

100 g des Trägermaterials wurden mit 140 ml einer wäßrigen Lösung von 0,95 g SnCl₂ · 2H₂O, 1,05 g In(NO₃)₃ und 6,03 g LiNO₃ imprägniert. Die Lösung enthielt darüber hinaus 1,2 Gew.-% HNO₃. Nach der Imprägnierung wurde das Trägermaterial getrocknet und für die Dauer von 1,5 Stunden bei 550°C calciniert. Anschließend wurde das Trägermaterial mit 140 ml einer wäßrigen Lösung imprägniert die 0,4 g Pt in der Form von H₂PtCl₆ und 1,2 Gew.-% HNO₃ enthielt. Die so erhaltene Katalysatorvorstufe wurde erneut wie schon oben beschrieben getrocknet und calciniert. Abschließend wurde der Katalysator bei 490°C in einem Strom aus Formiergas (5 Vol.-% H₂; 95 Vol.-% N₂) reduziert. Der fertige Katalysator enthielt 0,4 Gew.-% Platin, 0,5 Gew.-% Zinn, 0,4 Gew.-% Indium und 0,6 Gew.-% Lithium.

Dieser Katalysator und auch die Katalysatoren der folgenden Beispiele wurden für die Dehydrierung eines C₁₀- bis C₁₄-Paraffingemisches eingesetzt. Dazu wurden jeweils 10 ml des Katalysators in einen Reaktor gefüllt, der von einem Gasgemisch aus Wasserstoff und Paraffinen bei einem Druck von 2,3 bar und einer LHSV von 22 h⁻¹ durchgespült wurde. Das Verhältnis H₂/Paraffin betrug 4,7 mol/mol. Die Anfangstemperatur im Katalysatorbett wurde so gewählt, daß im Flüssigprodukt ein Olefingehalt von ca. 11 Gew.-% erreicht wurde. Dies war bei einer Temperatur von 453°C der Fall. Da der Katalysator desaktivierte, mußte im Laufe des Tests die Temperatur regelmäßig angehoben werden. Nach 7,5 Tagen war eine Katalysatortemperatur von 490°C erreicht. Der Durchschnittsolefingehalt im Flüssigprodukt lag bei diesem Test bei 11,2 Gew.-%.

### Beispiel 1

In gleicher Weise wie der konventionelle Katalysator wurde ein erfindungsgemäßer Katalysator B hergestellt. LiNO₃ wurde jedoch durch entsprechende Mengen Mg(NO₃)₂ · 6H₂O ersetzt. Der fertige Katalysator enthielt 0,4 Gew.-% Platin, 0,5 Gew.-% Zinn, 0,4 Gew.-% Indium und 2,1 Gew.-% Magnesium.

Auch dieser Katalysator wurde wie der Katalysator von Vergleichsbeispiel 1 für die Dehydrierung eines C₁₀- bis C₁₄-Paraffingemisches eingesetzt. Der Durchschnittsolefingehalt betrug bei diesem Test 11,8 Gew.-%. Die Endtemperatur von 490°C wurde erst nach 10 Tagen erreicht. Der erfindungsgemäße Katalysator B ist also viel stabiler als der konventionelle Katalysator A. Mit dem Katalysator B wurde eine um ein Drittel höhere Laufzeit erreicht, obwohl der Durchschnittsgehalt des Olefins um 0,6 Gew.-% höher lag als bei Katalysator A.

### Beispiel 2

Ein erfindungsgemäßer Katalysator C wurde in einem einstufigen Verfahren durch gleichzeitige Aufbringung aller Katalysatorkomponenten aus einer gemeinsamen Lösung auf den Katalysatorträger hergestellt.

Dazu wurden 100 g des Trägermaterials von Vergleichsbeispiel 1 mit 140 ml einer wäßrigen Lösung von 0,79 g Pt(NH₃)₄(NO₃)₂, 22,39 g Mg(NO₃)₂ · 6H₂O, 1,05 g In(NO₃)₃ und 0,95 g SnCl₂ · 2H₂O imprägniert. Die Lösung enthielt darüber hinaus 1,2 Gew.-% HNO₃. Die so erhaltene Katalysatorvorstufe wurde wie in Vergleichsbeispiel 1 beschrieben getrocknet, calciniert und reduziert. Dieser durch einstufige Imprägnierung hergestellte Katalysator C wies eine ausgezeichnete Langzeitstabilität auf. Die Endtemperatur von 490°C wurde erst nach 22 Tagen erreicht.

### Beispiel 3

Es wurde ein weiterer erfindungsgemäßer Katalysator D wie in Beispiel 2, jedoch ohne Indium hergestellt und für die Dehydrierung von C₁₀- bis C₁₄-Paraffinen eingesetzt. Der durchschnittliche Olefingehalt bei diesem Test betrug 10,9 Gew.-%. Nach 16 Tagen wurde eine Katalysatortemperatur von 490°C erreicht. Obwohl dieser Katalysator kein Indium enthielt, lieferte er deutlich größere Mengen an Olefin als der konventionelle Katalysator A mit Indium.

### Vergleichsbeispiel 2

Ein Vergleichskatalysator E wurde auf Basis eines Aluminiumoxidträgers, der ein Schüttgewicht von 0,6 g/cm³ und ein Porenvolumen von 0,64 ml/g aufwies, hergestellt. 100 g dieses Trägers wurde mit 0,3 g Pt(NH₃)₄(NO₃)₂, 0,47 g In(NO₃)₃, 22,38 g Mg(NO₃)₂ · 6H₂O und 0,43 g SnCl₂ · 2H₂O, gelöst in 64 ml einer 1,2 Gew.-% HNO₃-Lösung, imprägniert, getrocknet, calciniert und reduziert bei 490°C. Die Metall-Gehalte waren Pt: 0,18; In: 0,18; Sn: 0,23 und Mg: 2,1 Gew.-%.

10 ml (5,93 g) dieses Katalysators wurde genau wie im Vergleichsbeispiel 1 beschrieben für die Dehydrierung getestet. Der im Vergleich zu den anderen Beispielen niedrigere Pt-Gehalt des Katalysators wurde genau durch dessen höheres Schüttgewicht kompensiert. Dementsprechend war die Menge des katalytisch aktiven Platins in jeder der hier aufgeführten Anwendungsbeispiele identisch.

Bei einer Anfangstemperatur von 462°C war der Olefingehalt im Produktstrom mit lediglich 4,9 Gew.-% eindeutig zu niedrig, eine Temperaturerhöhung auf 481°C brachte hierin keine Verbesserung. Die schlechten Ergebnisse dieses Katalysators sind auf seine geringe Porosität zurückzuführen, die den Zugang der Reaktanden zu den katalytisch aktiven Komponenten erschwert.

## Patentansprüche

1. Katalysator für die Dehydrierung von C₆- bis C₁₅-Paraffinen, enthaltend auf einem anorganischen Träger 0,01 bis 5 Gew.-% mindestens eines Platingruppenmetalls, 0,01 bis 5 Gew.-% mindestens eines der Elemente Zinn, Germanium und Blei als Promotoren sowie Magnesium als zusätzlichen Modifikator,
mit einem Gewichtsanteil am fertigen Katalysator von 0,01 bis 20 Gew.-% wobei der Träger ein Schnittgewiche von 0,25 bis überhalb von 0,5 g/cm³ aufweisen.

2. Katalysator nach Anspruch 1,
**dadurch gekennzeichnet**,
daß es sich bei dem Platingruppenelement um Platin handelt.

3. Katalysator nach einem der Ansprüche 1 bis 2,
**dadurch gekennzeichnet**,
daß es sich bei dem Promotor um Zinn handelt.

4. Katalysator nach einem der Ansprüche 1 bis 3,
**dadurch gekennzeichnet**,
daß der Katalysator noch bis 5 Gew.-% Indium enthält.

5. Katalysator nach einem der Ansprüche 1 bis 4,
**dadurch gekennzeichnet**,
daß der Katalysator noch bis 5 Gew.-% Schwefel enthält.

6. Verfahren zur Herstellung des Katalysators nach Anspruch 1,
**gekennzeichnet durch**
gleichzeitiges Einbringen von löslichen Vorstufen der Platingruppenmetalle, der Promotoren und des Modifikators aus einer gemeinsamen Imprägnierlösung in den Träger, Trocknen des Trägers, Calcinieren bei Temperaturen zwischen 300 und 700°C und abschließendes Reduzieren in einem Wasserstoff enthaltenden Gas bei Temperaturen zwischen 200 und 700°C, wobei als Modifikator Magensium mit einem Gewichtsanteil von 0,01 bis 20 Gew.-% am fertigen Katalysator eingesetzt wird.

7. Verfahren nach Anspruch 6,
**dadurch gekennzeichnet**,
daß die gemeinsame Imprägnierlösung noch lösliche Vorstufen von Indium enthält.

8. Verfahren nach einem der Ansprüche 6 und 7,
**dadurch gekennzeichnet**,
daß die gemeinsame Imprägnierlösung noch lösliche Vorstufen von Schwefel enthält.

9. Verfahren zur Dehydrierung von C₆- bis C₁₅-Paraffinen,
**dadurch gekennzeichnet**,
daß die Dehydrierung an einem Katalysator nach einem der Ansprüche 1 bis 5 durchgeführt wird.

## Claims

1. A catalyst for the dehydrogenation of C₆-C₁₅-paraffins containing, on an inorganic support, 0.01 to 5 wt.% of at least one platinum group metal, 0.01 to 5 wt.% of at least one of the elements tin, germanium and lead as promoters and magnesium as an additional modifier with a concentration by weight in the final catalyst of 0.01 to 20 wt.%, wherein the support has a bulk density of 0.25 to 0.5 g/cm³.

2. A catalyst according to Claim 1, characterised in that the platinum group element is platinum.

3. A catalyst according to one of Claims 1 to 2, characterised in that the promoter is tin.

4. A catalyst according to one of Claims 1 to 3, characterised in that the catalyst also contains up to 5 wt.% of indium.

5. A catalyst according to one of Claims 1 to 4, characterised in that the catalyst also contains up to 5 wt.% of sulfur.

6. A process for preparing a catalyst according to Claim 1 by simultaneous introduction to the support of soluble precursors of the platinum group metals, promoters and modifier from a common impregnation solution, drying of the support, calcining at temperatures between 300 and 700°C and then reducing in a hydrogen-containing gas at temperatures between 200 and 700°C, wherein magnesium as modifier is used at a concentration by weight of 0.01 to 20 wt.% of the final catalyst.

7. A process according to Claim 6, characterised in that the common impregnation solution also contains soluble precursors of indium.

8. A process according to one of Claims 6 and 7, characterised in that the common impregnation solution also contains soluble precursors of sulfur.

9. A process for the dehydrogenation of C₆-C₁₅-paraffins, characterised in that the dehydrogenation is performed on a catalyst according to one of Claims 1 to 5.

## Revendications

1. Catalyseur pour la déshydrogénation des paraffines en C₆ à C₁₅ renfermant sur un support minéral, de 0,01 à 5 % en poids d'au moins un métal du groupe du platine, de 0,01 à 5 % en poids d'au moins un des éléments choisis parmi l'étain, le germanium, et le plomb, en tant que promoteur ainsi que le magnésium en tant que modificateur supplémentaire, avec une proportion pour le catalyseur terminé, de 0,01 à 20 % en poids, pour lequel le support possède une densité apparente de 0,25 à moins de 0,5 g/cm³.

2. Catalyseur selon la revendication 1,
caractérisé en ce qu'
en ce qui concerne l'élément du groupe du platine, il s'agit du platine.

3. Catalyseur selon l'une des revendications 1 à 2,
caractérisé en ce qu'
en ce qui concerne le promoteur, il s'agit de l'étain.

4. Catalyseur selon l'une des revendications 1 à 3,
caractérisé en ce que
le catalyseur renferme encore jusqu'à 5 % en poids d'indium.

5. Catalyseur selon l'une des revendications 1 à 4,
caractérisé en ce que
le catalyseur renferme encore jusqu'à 5 % en poids de soufre.

6. Procédé de production du catalyseur selon la revendication 1,
caractérisé par
l'introduction simultanée de stades préliminaires solubles de métaux du groupe du platine, de promoteurs et du modificateur à partir d'une solution d'imprégnation commune dans le support, séchage du support, calcination à des températures comprises entre 300 et 700°C et réduction finale dans le gaz contenant de l'hydrogène à des températures comprises entre 200 et 700°C dans lequel comme modificateur on met en oeuvre du magnésium avec un pourcentage en poids de 0,01 à 20 % en poids sur le catalyseur terminé.

7. Procédé selon la revendication 6,
caractérisé en ce que
la solution d'imprégnation commune renferme encore des stades préliminaires solubles d'indium.

8. Procédé selon l'une des revendications 6 et 7,
caractérisé en ce que
la solution d'imprégnation commune renferme encore des stades préliminaires solubles de soufre.

9. Procédé de déshydrogénation de paraffines en C₆ à C₁₅.
caractérisé en ce que
la déshydrogénation est effectuée sur un catalyseur selon l'une des revendications 1 à 5.
